# EUROPEAN PATENT APPLICATION

(11) **EP 4 738 248 A1**
(43) Date of publication of application: **06.05.2026**
(21) Application number: 25207040.4
(22) Date of filing: 06.10.2025
(51) Int. Cl.: G06T 7/10, G16H 15/00, G16H 30/40

(54) **SCHEMATIC REPRESENTATION OF A HEALTH STATE OF TISSUE OF INTEREST BASED ON MEDICAL IMAGE DATA**

(30) Priority: 29.10.2024 US 202418930845
(71) Applicant: GE Precision Healthcare LLC, Waukesha, WI 53188 (US)
(72) Inventor: MORARD, Vincent, Waukesha, 53188-1696 (US); LONDOÑO, Jorge Eduardo Hernandez, Waukesha, 53188-1696 (US); BOURGHES, Sofiène, Waukesha, 53188-1696 (US)
(74) Representative: Kilburn & Strode LLP

(57) **Abstract**

A computer-implemented method includes obtaining a first mask of primary tissue of interest segmented from image data of a subject, obtaining a second set of masks of other tissue indicative of the health state of the primary tissue of interest from the image data of the subject, obtaining a third set of masks of elements that are signs of disease of the primary tissue of interest from the image data of the subject, and generating a schematic representation of the health state of the primary tissue of interest of the subject with the image data, the first mask, the second set of masks and the third set of masks. In one instance, a health state of the primary tissue of interest is under evaluation and the schematic representation provides a summary of health related information about the health state of the primary tissue of interest from the image data.

## Description

### FIELD

The following generally relates to medical imaging, and more particularly to a schematic representation of a health state of tissue of interest that is based on medical image data.

### BACKGROUND

Clinicians such as radiologists "read" (i.e., interpret) images, e.g., for diagnosis, monitoring, screening, etc. For example, a clinician observes images in a study for a subject and documents findings (i.e., relevant observations), which include a description of abnormalities and/or normal notable features with respect to tissue, organs, bones, etc. For diagnosis, the findings assist the clinician with identifying diseases, injuries, abnormalities, etc. For monitoring, the findings may assist the clinician with understanding the progression of diseases, injuries, abnormalities, etc. For screening, the findings may assist the clinician with identifying conditions that are likely to lead to certain diseases, injuries, abnormalities, etc.

The literature indicates that early detection of diseases, injuries, abnormalities, etc. improves treatment outcomes, including, with respect to certain diseases, the survival rate of patients. Existing visualization approaches have included generating and displaying renderings (e.g., mesh, volume, etc.) and using generic atlases or the like to visualize tissue under evaluation. Unfortunately, such approaches have poorly characterized a health state of the tissue under evaluation and/or other tissue in connection detection of diseases, injuries, abnormalities, etc. and/or conditions likely to lead thereto. In view of the foregoing, there is an unresolve need for an improved approach.

### SUMMARY

Aspects described herein address the above-referenced problems and others. This summary introduces concepts that are described in more detail in the detailed description. It should not be used to identify essential features of the claimed subject matter, nor to limit the scope of the claimed subject matter.

In one aspect, a computer-implement method includes obtaining a first mask of primary tissue of interest segmented from image data of a subject. In one instance, a health state of the primary tissue of interest is under evaluation. The computer-implement method further includes obtaining a second set of masks of other tissue indicative of the health state of the primary tissue of interest from the image data of the subject. The computer-implement method further includes obtaining a third set of masks of elements that are signs of disease of the primary tissue of interest from the image data of the subject. The computer-implement method further includes generating a schematic representation of the health state of the primary tissue of interest of the subject with the image data, the first mask, the second set of masks and the third set of masks. In one instance, the schematic representation provides a summary of health related information about the health state of the primary tissue of interest from the image data.

In another aspect, a system includes a display device, a memory, and at least one processor. The memory is configured to store computer-readable instructions for generating a schematic representation of a health state of a primary tissue of interest of a subject based on image data of the subject. The at least one processor is configured to execute the computer-readable instructions. The computer-readable instructions cause the at least one processor to obtain a first mask of the primary tissue of interest segmented from the image data, obtain a second set of masks of other tissue indicative of the health state of the primary tissue of interest from the image data of the subject, obtain a third set of masks of elements that are signs of disease of the primary tissue of interest from the image data of the subject, and generate the schematic representation with the image data, the first mask, the second set of masks and the third set of masks. The schematic representation provides a summary of health related information about the health state of the primary tissue of interest from the image data.

In another aspect, a computer readable medium is encoded with computer executable instructions. The computer executable instructions, when executed by a processor, cause the processor to obtain a first mask of primary tissue of interest segmented from image data of a subject, wherein a health state of the primary tissue of interest is under evaluation, obtain a second set of masks of other tissue indicative of the health state of the primary tissue of interest from the image data of the subject, obtain a third set of masks of elements that are signs of disease of the primary tissue of interest from the image data of the subject, and generate a schematic representation of the health state of the primary tissue of interest of the subject with the image data, the first mask, the second set of masks and the third set of masks. The schematic representation provides a summary of health related information about the health state of the primary tissue of interest from the image data.

Those skilled in the art will recognize still other aspects of the present application upon reading and understanding the attached description.

### BRIEF DESCRIPTION OF THE DRAWINGS

The application is illustrated by way of example and not limited by the figures of the accompanying drawings in which like references indicate similar elements.
FIGURE 1 diagrammatically illustrates a non-limiting example of a system that includes a schematic representation module, in accordance with an aspect of an embodiment(s) herein.
FIGURE 2 diagrammatically illustrates a non-limiting example of the schematic representation module, in accordance with an embodiment(s) herein.
FIGURE 3 diagrammatically illustrates a non-limiting example of an image data selector of the schematic representation module, in accordance with an embodiment(s) herein.
FIGURE 4 diagrammatically illustrates a non-limiting example of filter criteria for the image data selector, in accordance with an embodiment(s) herein.
FIGURE 5 diagrammatically illustrates another non-limiting example of filter criteria for the image data selector, in accordance with an embodiment(s) herein.
FIGURE 6 diagrammatically illustrates a variation of the image data selector, in accordance with an embodiment(s) herein.
FIGURE 7 diagrammatically illustrates a non-limiting example of a segmentation mask generator of the schematic representation module, in accordance with an embodiment(s) herein.
FIGURE 8 diagrammatically illustrates a non-limiting example of trained segmentation mask generator, in accordance with an embodiment(s) herein.
FIGURE 9 diagrammatically illustrates a non-limiting example of a feature determiner of the schematic representation module, in accordance with an embodiment(s) herein.
FIGURE 10 diagrammatically illustrates a non-limiting example of a schematic representation generator of the schematic representation module, in accordance with an embodiment(s) herein.
FIGURE 11 diagrammatically illustrates a non-limiting example of a schematic representation, in accordance with an embodiment(s) herein.
FIGURE 12 diagrammatically illustrates another non-limiting example of a schematic representation, in accordance with an embodiment(s) herein.
FIGURE 13 diagrammatically illustrates a non-limiting example of an imaging system configured for CT imaging, in accordance with an embodiment(s) herein.
FIGURE 14 illustrates a non-limiting example of a flow chart for generating a schematic representation that provides a visual summary of a health state of tissue of interest, in accordance with an aspect herein
FIGURE 15 illustrates a non-limiting example of a flow chart for generating a schematic representation that provides a visual summary of a health state of the liver for a particular subject, in accordance with an aspect herein.

### DETAILED DESCRIPTION

Embodiments of the present disclosure will now be described, by way of example, with reference to the figures, in which a system, a method and/or instructions of a computer readable medium generate a schematic representation of a health state of primary tissue of interest of a subject. The schematic representation can be generated with CT, MR, SPECT, PET, and/or other image data of the subject. In one instance, the approach includes generating and/or obtaining masks for the primary tissue of interest, other tissue indicative of a health state of the primary tissue of interest and elements of signs of disease of the primary tissue of interest. In some instance, the approach further includes determining and/or obtaining classified metrics indicative of a health state of the primary tissue of interest and including the classified metrics with the schematic representation. In some instance, the approach further includes utilizing the image data to generate renderings that can be displayed along with the schematic representation.

For a trained clinician such as a radiologist, a few seconds of examining the schematic representation is likely enough to determine whether further analysis is required. This schematic representation can also be included in clinical reports, e.g., to share with other radiologists or to summarize studies, facilitating communication and collaboration. The schematic representation can consider both primary and secondary signs of disease in primary tissue of interest, and searches for indications of the disease in other organs and/or other areas. In one instance, the approach can be used to assist screening. This includes processes image data acquired for reasons unrelated to the reason for imaging the primary tissue of interest, potentially triggering further analysis and early disease detection. The approach can also be used for assisting with diagnoses. The approach can also be used for assisting with monitoring. Other applications are also contemplated herein.

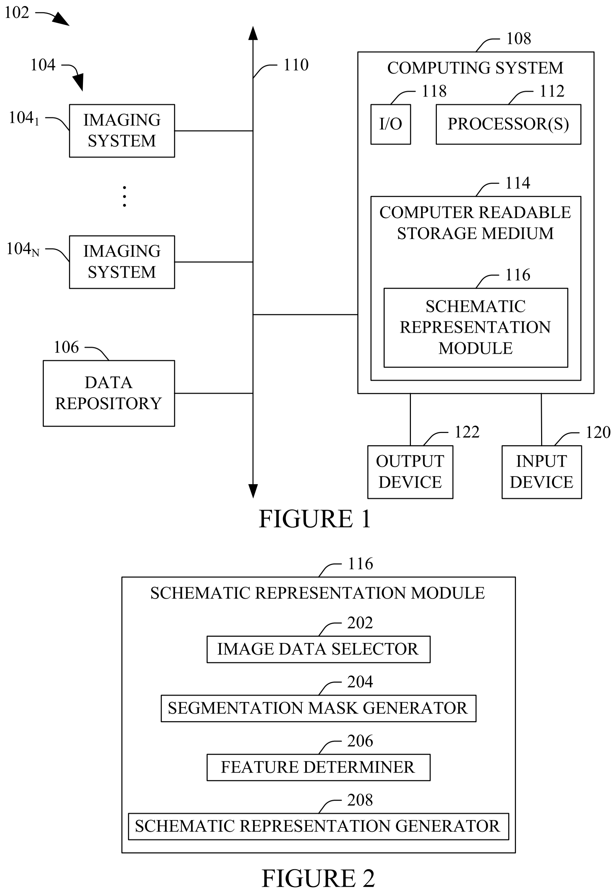

Initially referring to FIGURE 1, a system 102 including a set of medical imaging modalities 104, a data repository 106 and a computing system 108 is diagrammatically illustrated. The set medical imaging modalities 104, the data repository 106 and the computing system 108 are in communication with a network 110. Such communication can be through wired and/or wireless technologies. In one instance, the network 110 is configured for communicating medical data, including image data and non-image data. In one instance, the network 110 is configured for communicating image data using file formats such as Digital Imaging and Communications in Medicine (DICOM), and/or other format, and non-image data using protocols such as Health Level Seven (HL7) and/or other protocol. The set of medical imaging modalities 104, the data repository 106 and the computing system 108 can communicate with each other and/or other devices via the network 110.

In the illustrated example, the set of medical imaging modalities 104 includes a first imaging system 104₁, ..., and an Nth imaging system 104_{N}, where N is an integer equal to or greater than one. Examples of imaging systems of the set of medical imaging modalities 104 include one or more Computed Tomography (CT) imaging systems, one or more Magnetic Resonance (MR) imaging systems, one or more X-ray imaging systems, one or more Positron Emission Tomography (PET) imaging systems, one or more Single Photon Emission Computed Tomography (SPECT) imaging systems, etc. The set of medical imaging modalities 104 may include imaging systems in connection with a single entity such as a hospital, an imaging center, etc., or imaging systems in connection with multiple different entities.

As described in greater detail below, the imaging systems are configured to generate volumetric image data. In some instances, at least one of the imaging systems of the set of medical imaging modalities 104 is configured to push and/or pull image data over the network 110, e.g., to and/or from the data repository 106 and/or the computing system 108. In some instances, at least one of the imaging systems of the set of medical imaging modalities 104 is configured to receive image data over the network 110, e.g., from the data repository 106 and/or the computing system 108. In some instances, at least one of the imaging systems of the set of medical imaging modalities 104 is configured to allow access to image data stored therein over the network 110, e.g., by the data repository 106 and/or the computing system 108.

The data repository 106 includes one or more of a Radiology Information System (RIS), a server such as a Picture Archiving and Communications System (PACS) server, a Hospital Information System (HIS), an Electronic Medical Record (EMR), a database, and/or the like. The data repository 106 is configured to store image data and/or non-image data, such as image data generated by at least one of the imaging systems of the set of medical imaging modalities 104 and/or another imaging system, and/or image data processed by the computing system 108. DICOM fields of the image data include information such as a subject unique identification, an identification of the imaging modality utilized to acquire the image data, an identification of the anatomy under evaluation, an identification of the series included in the study, an identification of whether a contrast material was utilized for the acquisition, etc.

In some instances, at least one device of the data repository 106 is configured to push or pull image data and/or non-image data over the network 110, e.g., to or from the set of medical imaging modalities 104 and/or the computing system 108. In some instances, at least one device of the data repository 106 is configured to receive image data and/or non-image data over the network 110, e.g., from the set of medical imaging modalities 104 and/or the computing system 108. In some instances, at least one device of the data repository 106 is configured to allow access to image data and/or non-image data stored therein over the network 110, e.g., by the set of medical imaging modalities 104 and/or the computing system 108.

The computing system 108 includes a computer, a workstation (e.g., a PACS workstation configured with an image viewer, etc.), distributed processing services, cloud processing resources, etc. The computing system 108 includes at least one processor 112 such as a microprocessor (µP), a central processing unit (CPU), graphics processing unit (GPU), etc. The computing system 108 further includes a computer readable storage medium 114, which includes non-transitory medium and excludes transitory medium (signals, carrier waves, and the like). The at least one processor 112 is configured to execute software resident encoded or embedded on the computer readable storage medium 114. In the illustrated example, such software at least includes a schematic representation module 116.

As described in greater detail below, the schematic representation module 116 is configured to generate, from image data, segmentation masks, determined and classify metrics based on the image data and the masks, and generate a schematic representation of tissue of interest that summarizes a health state of the tissue of interest, without volume renderings, atlases, etc., although volume renderings, atlases, etc. may be included with the schematic representation. Again, in one instance the schematic representation provides a summary of a health state of the primary tissue of interest of a subject. In one instance, the schematic representation assists a reading clinician with interpreting the image data. For example, in one instance the approach at least assists with early detection of diseases, injuries, abnormalities, etc. and improves treatment outcomes, providing capabilities absent from approaches that omit the schematic representation module 116.

The computing system 108 further includes input/output (I/O) 118. An input device 120 includes a keyboard, mouse, touchscreen, microphone, etc. The input device 120 is in electrical communication with the computing system 108 through the I/O 118 and/or otherwise. An output device 122 includes a human readable device such as a display monitor or the like. The output device 122 is in electrical communication with the computing system 108 through the I/O 118 and/or otherwise.

In some instances, the computing system 108 is configured to push and/or pull image data and/or non-image data over the network 110, e.g., to and/or from the set of medical imaging modalities 104 and/or the data repository 106. In some instances, the computing system 108 is configured to receive medical data and/or non-image data over the network 110, e.g., from the set of medical imaging modalities 104 and/or the data repository 106. In some instances, the computing system 108 is configured to allow access to medical data and/or non-image data stored therein over the network 110, e.g., by the set of medical imaging modalities 104 and/or the data repository 106.

Turning to FIGURE 2, a non-limiting example of the schematic representation module 116 is diagrammatically illustrated. In this example, the schematic representation module 116 includes an image data selector 202, a segmentation mask generator 204, a feature determiner 206, and a schematic representation generator 208. In some instances, at least one of the medical data selector 202, the segmentation mask generator 204, and/or the feature determiner 206 is executed with different processing resources (i.e., not by the computing system 108). In such an instance, the schematic representation module 116 may or may not include the at least one of the image data selector 202, the segmentation mask generator 204, and/or the feature determiner 206.

The image data selector 202 obtains a set of image data from one or more sources of the data repository 106 for the segmentation mask generator 204 to process. In one instance, after the imaging system 104₁, ..., the MR imaging system 104_{N} of the set of imaging modalities 104 is utilized to scan a subject under evaluation and generate image data for the subject, the image data is stored in one or more sources of the data repository 106 as imaging study. In one instance, the image data is automatically stored in the source. In another instance, the image data is semi-automatically stored in the source, e.g., based on a user input. The image data selector 202 is configured to obtain the set of image data from the one or more sources of the data repository 106 based on user-defined, machine learned, etc. filter criteria.

In one instance, the medical data selector 202 is notified when an imaging study is stored in the data repository 106. For example, where the data repository 106 is a RIS, the RIS may automatically add the imaging study to a worklist for the schematic representation module 116, and the image data selector 202 is apprised of the imaging study by way of the worklist. In some instance, the act of storing image data in the data repository 106 invokes the medical image data selector 202 to access the imaging study. In another instance, the image data selector 202 accesses imaging studies in the data repository 106 when processing resources of the computing system 108 and/or other processing resources are available. In another instance, the image data selector 202 accesses imaging studies in the data repository 106 based on a predetermined schedule. In another instance, the image data selector 202 accesses imaging studies in the data repository 106 on-demand based on a user input.

The segmentation mask generator 204 is configured to process, with a set of algorithms, the set of image data obtained by the image data selector 202 to generate a set of masks. Examples of such masks include one or more segmentation masks of primary tissue of interest, one or more sub-segmentation masks of the one or more segmentation masks of the primary tissue of interest, other tissue relevant to a health state of the primary tissue of interest, elements indicative of signs of a disease of the primary tissue of interest, etc. The feature determiner 206 is configured to process the set of masks and determine and classify health related metrics for the primary tissue of interest based on measurements such as size, shape, etc. and known ranges for at least healthy tissue. The schematic representation generator 208 is configured to process the masks (and the classified metrics) and generate, based on a set of rules, a schematic representation of the primary tissue of interest (and the other tissue, the elements and the measurements) that summarizes a health state of the primary tissue of interest.

The schematic representation module 116 outputs the schematic representation, which can be displayed via a display monitor of the output device 122, incorporated into a radiological report for the study, stored in the computing system 108 and/or the data repository 106, etc. In one instance, the schematic representation is generated using a markup language based image format such as Extensible Markup Language (XML) based image format. As example of such a suitable XML based image format is Scalable Vector Graphics (SVG). Using such an XML-based vector image format allows for presenting the schematic representation without loss of quality, whether on a display monitor, in a report, etc. Although the creation and display of the schematic representation does not require volume renderings, atlases, etc., optionally, volume renderings, atlases, etc. may be included with the schematic representation.

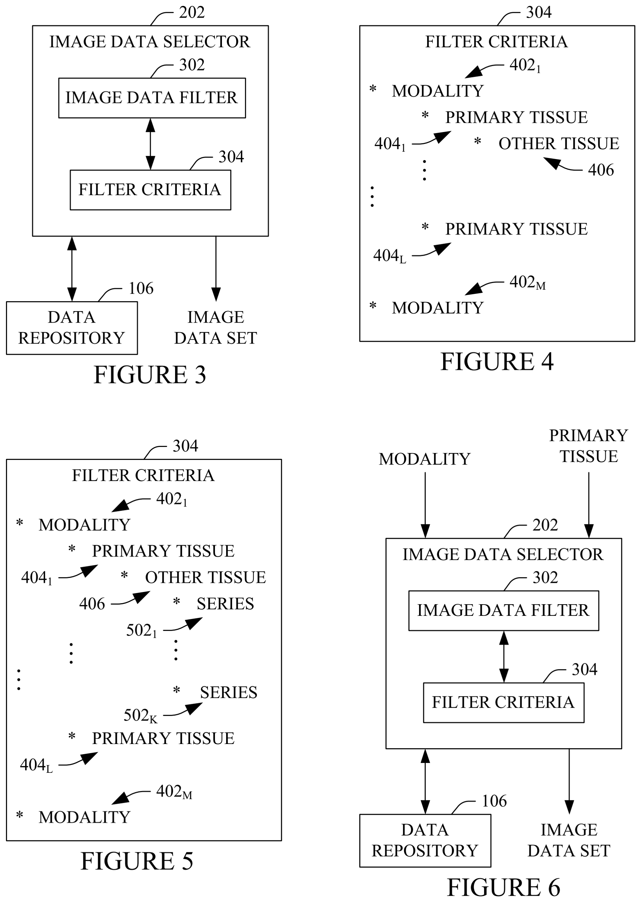

FIGURE 3 diagrammatically illustrates a non-limiting example of the image data selector 202. The image data selector 202 includes an image data filter 302 and filter criteria 304. The filter criteria 304 includes information such as an imaging modality of interest (e.g., CT, MR, X-ray, PET, SPECT, etc.), a primary tissue of interest (e.g., liver, heart, lungs, kidneys, spleen, gallbladder, etc.), tissue relevant to a health state of the primary tissue of interest (e.g., for the liver, the spleen, the kidneys, the pancreas, etc.), etc. The image data filter 302 filters the studies in the data repository 106 based on the filter criteria 304 to obtain a set of image data for processing. The image data selector 202 outputs the set of image data set.

FIGURE 4 diagrammatically illustrates a non-limiting example of the filter criteria 304. In this example, the filter criteria 304 includes M modalities, a modality 402₁, ... and a modality 402_{M} (collectively referred to herein as modalities 402), where M is an integer equal to or greater than one. Each of the M modalities includes a plurality of primary tissues of interest. For example, the modality 402₁ includes L tissues of interest, including primary tissue 404₁, ... and primary tissue 404_{L} (collectively referred to herein as primary tissues 404), where L is an integer equal to or greater than one. Each of the plurality of primary tissue identifies other tissue that is relevant to the health of the primary tissue. For example, the primary tissue 404₁ includes other tissue 406.

In one instance, the image data selector 202 would first filter the data repository 106 for studies corresponding to the modality 402₁. In one instance, the filtering includes searching a DICOM and/or other field of the files of the studies in the data repository 106. Within the set of studies corresponding to the modality 402₁, the image data selector 202 would filter the data repository 106 for studies corresponding to the primary tissue 404₁. Within the set of studies corresponding to the primary tissue 404₁, the image data selector 202 would filter the data repository 106 for studies including the other tissues 406.

The identified studies would be grouped together, but delineated e.g., by subject, using a unique identifier (UID) and/or other information. By way of non-limiting example, where the modality 402₁ is CT, the primary tissue 404₁ is the liver, and other tissues 406 include the spleen, the kidney and the pancreas, the image data selector 202 finds CT studies of the liver that also include the spleen, the kidney and the pancreas, groups them together, but separates them based on the subject. For example, in one instance the image data selector 202 identifies a CT abdomen study that includes the liver, the spleen, the pancreas and the kidney. The image data selector 202 repeats the above through the primary tissue 404_{L} for the modality 402₁. This process continues through the modality 402_{M}. The final set of image data is delineated based on the modality, on the primary tissue, and on the subject. As such, the image data can be processed based on a combination of the modality, the primary tissue and the subject for generation of a schematic representation for the primary tissue of the subject

FIGURE 5 diagrammatically illustrates another non-limiting example of the filter criteria 304. In this variation, the filter criteria 304 further includes series identification, including series 502₁, ..., and series 502_{K} (collectively referred to herein as series 502), where K is an integer equal to or greater than one. In one instance, the series 502 provides another level of filtering. Continuing with the example discussed in connection with FIGURE 4, in one instance the series 502₁, ..., 502_{K} may indicate series images acquired during a contrast material enhanced scan, such as images around a particular state such as a particular contrast phase of blood vessels such as a non-enhanced phase, the arterial phase, the portal phase, the delayed phase, etc. In another instance, the series 502 may indicate series acquired during different functional states such as a cardiac phase, a respiratory cycle, etc. Other levels of filtering are contemplated herein. For example, with MR image data, the filtering may include different sequences such T1, T2, Diffusion Weighted (DW), Fluid-Attenuated Inversion Recovery (FLAIR), Time of Flight (TOF), etc.

FIGURE 6 diagrammatically illustrates a variation of the image data selector 202 discussed in connection with FIGURE 3. In this variation, certain filter criteria such as the modality of interest and the primary tissue are provided to the image data selector 202. For example, a user of the computing system 108 can enter such information via the input device 118. The image data selector 202 would search the filter criteria 304 for the entered modality of interest and the primary tissue and filter the studies in the data repository 106 based on the entry in the filter criteria 304 that includes the entered modality of interest and primary tissue. In yet another instance, a user may enter the modality of interest, the primary tissue, the other tissues, the series, and/or other information. In such an instance, the image data selector 202 may not utilize the filter criteria 304. Alternatively, the image data selector 202 may determine whether there is any term in the filter criteria 304 not provided in the input, and display a notification indicating the term and/or a pop-up window or the like allowing the user to accept or reject adding the term through a mouse click or the like.

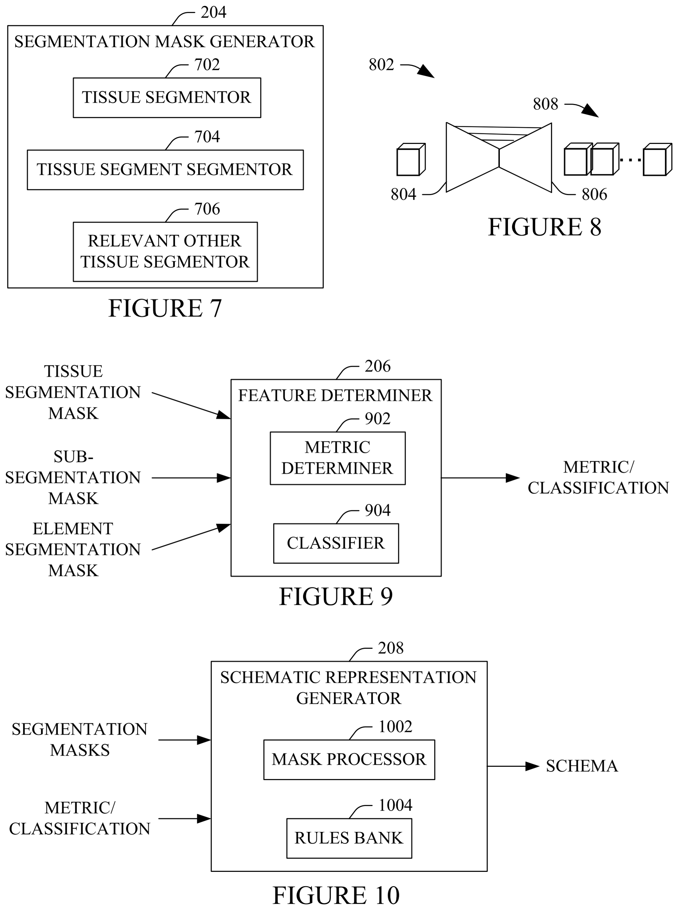

Next at FIGURE 7, a non-limiting example of the segmentation mask generator 204 is diagrammatically illustrated. In the illustrated example, the segmentation mask generator 204 includes a tissue segmentor 702, a tissue segment segmentor 704, and a relevant other tissue segmentor 706. The segmentation mask generator 204 processes the studies from the image data selector 202 and each of the tissue segmentor 702, the tissue segment segmentor 704, and the relevant other tissue segmentor 706 outputs a corresponding segmentation mask. In one instance, the tissue segmentor 702 segments the primary tissue and the other tissue relevant to the health of the primary tissue and creates masks for each, the tissue segment segmentor 704 segments the segment of the primary tissue into sub-segments, and the relevant other tissue segmentor 706 segments elements that are signs of disease of the primary tissue.

The tissue segmentor 702, the tissue segment segmentor 704, and the relevant other tissue segmentor 706 can utilize known and/or other segmentation approaches. For example, in one instance at least one of the tissue segmentor 702, the tissue segment segmentor 704, and the relevant other tissue segmentor 706 employs an artificial intelligence (AI) based approach such as a Neural Network (NN), e.g., a Convolutional Neural Network (CNN) like U-Net, etc. By way of non-limiting example, the tissue segmentor 702, the tissue segment segmentor 704, and the relevant other tissue segmentor 706 can employ different U-Net models that are trained to segment various parts of the image. An example U-Net model 802 is illustrated in FIGURE 8. The U-Net models 802 include an encoder 804, a decoder 806, and multichannel outputs to outcome multiple segmentations 808 with at least one inference.

In general, the particular model utilized by the segmentation mask generator 204 will depend on the modality and primary tissue of interest. Examples of suitable models include, but are not limited to, a seeded watersheed algorithm, a graph-cut method, an atlas-based segmentation, a random forest, a Support Vector Machine (SVM), a fully convolutional network, a vision transformer, etc. Other suitable approaches include edge detection such as search, zero-crossing and/or other edge detection techniques that find edges, thresholding, which employs a threshold value(s) to turn a gray-scale image into a binary image, etc.

Next at FIGURE 9, a non-limiting example of the feature determiner 206 is diagrammatically illustrated. The feature determiner 206 receives, as input, the segmentation masks produced and output by the image data selector 202. The feature determiner 206 includes a metric determiner 902 and a classifier 904. The metric determiner 902, based on the image data and/or the masks, determines measurements such as size, shape, etc. of different parts of the primary tissue, the other tissue, etc. The metric determiner 902 further defines metrics based on the measurements. For example, a metric may include determining a value based on a measurement for part (e.g., one or more sub-segments) of the primary tissue to a measurement of the primary tissue. The classifier 904 compares the values of the metrics to values or ranges of values that correspond to healthy (and/or non-healthy) tissue and classifies the metrics according to the values or ranges. The feature determiner 206 outputs the metrics with the classifications.

Next at FIGURE 10, a non-limiting example of the schematic representation generator 208 is diagrammatically illustrated. The schematic representation generator 208 receives, as input, the segmentation masks and the classified metrics. The schematic representation generator 208 includes a mask processor 1002 and a rules bank 1004. The mask processor 1002 generates the schematic representation based on rules in the rules bank 1004, which determines what data from the image data and/or masks to include in the schematic representation. In one instance, the summary includes a graphical representation of the primary tissue of interest and the relevant other tissue generated from the image data. The location and size of the primary tissue of interest and the relevant other tissue will reflect the location and size of the primary tissue of interest for a particular slice/image of interest of the image and/or an accumulation of locations and sizes of the relevant other tissue derived from a set of slices/images in a window around the particular slice/image. In some instances, the schematic representation further includes two-dimensional (e.g., length, width, diameter, etc.) and/or three-dimensional (e.g., volume, etc.) measurements for tissue. In some instances, the schematic representation further includes the derived and classified metrics.

The following describes an example use-case scenario. In this use-case scenario, the imaging modality is CT or MR, the primary tissue of interest is the liver, the other tissue includes at least the spleen and kidneys, and the series includes acquisition times prioritizing the portal phase. In general, liver disease accounts for over two million deaths annually, including those from cirrhosis, viral hepatitis, and liver cancer. The early stages of liver disease are usually asymptomatic, leading to delayed clinical care. Early detection of the disease tends to improve the survival rate of patients.

Fibrosis, characterized by the formation of scar tissue, leads to progressive thickening and stiffening of the liver tissue. This can have multiple consequences, such as portal hypertension (spleen enlargement, portal vein enlargement, and presence of ascites), changes in liver morphology (atrophy of segment 4, hypertrophy of the left liver, presence of surface liver nodularity, and right hepatic notches), or liver parenchyma heterogeneity. The approach described herein employs the image data to generate a schematic representation that can facilitate assessment of a health state of the liver, e.g., in cases of Advanced Chronic Liver Disease (ACLD).

The image data selector 202 of the segmentation mask generator (FIGURES 2-6) accesses at least one storage source of the data repository 106 (or the set of imaging modalities 104, the computer readable storage medium 112 and/or other storage) and identifies contrast enhanced image studies of the liver that include the spleen and kidneys and cover the portal phase. The tissue segmentor 702 of the segmentation mask generator 204 (FIGURES 2 and 7) processes the identified image data and segments the liver, the spleen and the kidneys from the identified image studies, and generates masks indicative thereof.

The tissue segment segmentor 704 of the segmentation mask generator 204 (FIGURES 2 and 7) segments the liver and generates a mask thereof. In this example, the tissue segment segmentor 704 is configured to segment the liver based on the Couinaud segmentation approach. As such, the tissue segment segmentor 704 segments the liver into eight functionally independent segments, each segment having its own vascular inflow, outflow and biliary drainage, with a center of each segment having a branch of the portal vein, hepatic artery and bile duct. The relevant other tissue segmentor 706 of the segmentation mask generator 204 (FIGURES 2 and 7) processes the identified image data and segments elements that are signs of liver disease, and generates masks indicative thereof. Examples of such tissue include the right hepatic notch, portal veins, the gallbladder, liver surface nodularity, ligament falciform, collateral vessels, ascites, perihilar portal space, etc.

The feature determiner 206 of the schematic representation module 116 (FIGURES 2 and 9) processes the masks and derives and classifies metrics indicative of a health state of the liver. Examples of such metrics include liver volume, left liver hypertrophy, segment 4 atrophy, segment 1 hypertrophy, liver surface modularity, perihilar space enlargement, enlargement of the ligament, enlargement of the spleen, enlargement of the portal vein, ascites presence, gallbladder wall thickening and liver heterogeneity, etc. For example, a left liver hypertrophy metric can be derived by a ratio of the Couinaud segments 1 and 2 to the total liver volume. The feature determiner 206 can classify the left liver hypertrophy metric by comparing the value of the ratio to one or more known ranges to classify the value as "within" or "outside" of a normal range. In some instances, the abnormal range is further delineated into quantized ranges, e.g., into "low," "mild," "severe," etc., or on a continuum.

The schematic representation generator 208 of the schematic representation module 116 (FIGURES 2 and 10) processes the image data, the masks and the metrics and generates a schematic representation that summarizes a health of the primary tissue based on the information in the image data, the masks and the metrics. The schematic representation can be customized by a user to effectively report the information for better visualization. An example of a set of rules from the rules bank 1004 (FIGURE 10) includes:
* determine a shape of the liver from a slice of the image data where the portal vein enters the liver;
* determine a shape of body at the slice;
* determine a shape of the spleen from a slice of the image data where the spleen is at its largest, e.g., based on a largest diameter of the spleen;
* accumulate each of the right hepatic notch, ligament, portal vein, collateral vessels and perihilar space segmentations along a z-plane through the image data, and
* accumulate each of the ascites, gallbladder and liver surface nodules along the z-plane about a predetermined slice margin around the slice.

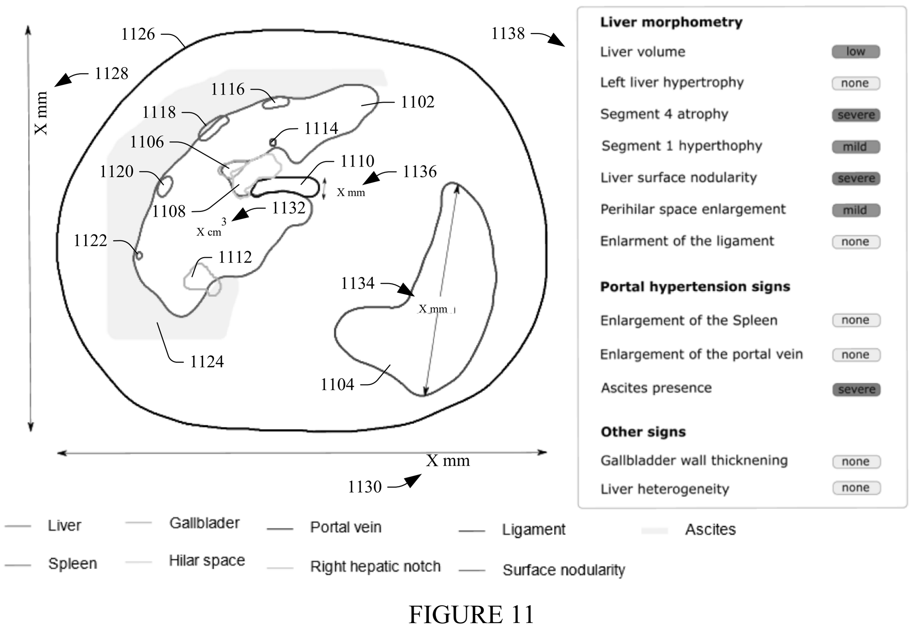

The schematic representation generator 208 incorporates the determined and accumulated information to generate the schematic representation, optionally along with a legend and metrics with their classifications. An example of such a schematic representation is diagrammatically illustrated in FIGURE 11. The schematic representation of FIGURE 11 can be visually presented in a graphical user interface (GUI) with a display monitor and/or otherwise, incorporated into a radiological report, etc.

In FIGURE 11, the schematic representation includes a liver 1102, a spleen 1104, a gallbladder 1106, a hilar space 1108, a portal vein 1110, a right hepatic notch 1112, a ligament 1114, a surface nodularity 1116, surface nodularity 1118, surface nodularity 1120, surface nodularity 1122, ascites 1124, and a contour of the body 1126. The schematic representation further includes a y-axis (depth) dimension 1128 and an x-axis (width) dimension 1130 for the contour of the body 1126, a volume 1132 of the liver 1102, a longest dimension 1134 of the spleen 1104, and a diameter 1136 of the portal vein 1110. Other and/or different information can be incorporated into other schematic representations.

The schematic representation further includes a set 1138 of the derived metrics along with corresponding classifications. In this example, the derived metrics are delineated under Liver morphometry, Portal hypertension signs and Other signs. The Liver Morphometry grouping includes Liver volume, Left liver hypertrophy, Segment 4 atrophy, Segment 1 hypertrophy, Liver surface modularity, Perihilar space enlargement and Enlargement of the ligament. The Portal hypertension signs grouping includes Enlargement of the spleen, Enlargement of the portal vein and Ascites presence. The Other signs grouping includes Gallbladder wall thickening and Liver heterogeneity. In other examples, a different set of derived metrics may be included in the schema.

In this example, the Liver volume feature is classified as low, the Left liver hypertrophy feature is classified as none, the Segment 4 atrophy feature is classified as severe, the Segment 1 hypertrophy feature is classified as mild, the Liver surface modularity feature is classified as severe, the Perihilar space enlargement feature is classified as mild, the Enlargement of the ligament feature is classified as none, the Enlargement of the spleen feature is classified as none, the Enlargement of the portal vein feature is classified as none, the Ascites presence feature is classified as severe, the Gallbladder wall thickening feature is classified as none, and the Liver heterogeneity feature is classified as none.

In this example, a gray scale coding is utilized for the classifications. A lightest shade of gray corresponds to the classification of none, a darker shade of gray corresponds to the classification of low, a next darker shade of gray corresponds to the classification of mild, and a darkest shade of gray corresponds to the classification of severe. Other coding schemes are contemplated herein. For example, in another instance the different classifications are presented via one or more of different font styles, colors, sizes, effects, patterns, etc.

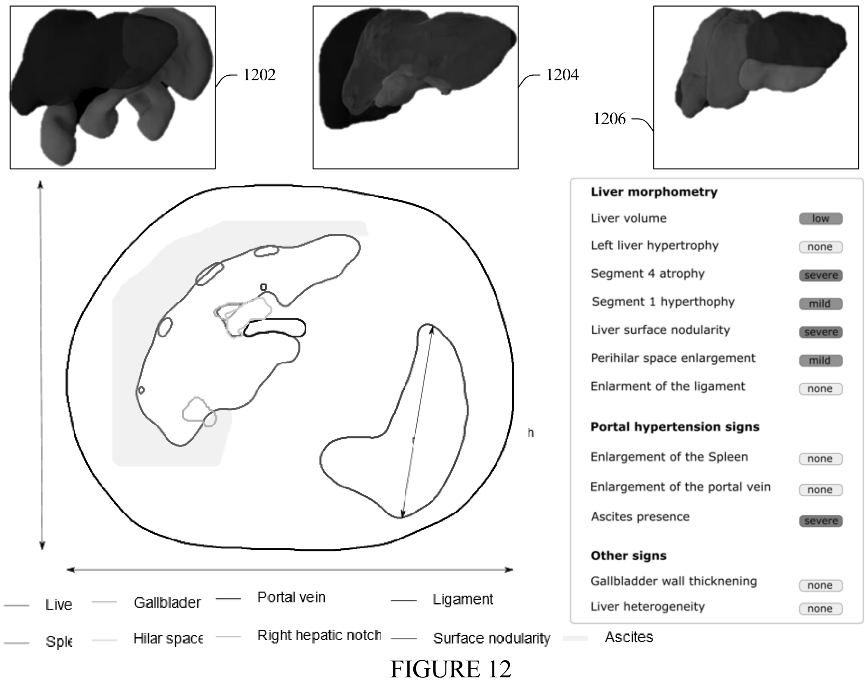

FIGURE 12 diagrammatically illustrates a variation of the schematic representation discussed in connection with FIGURE 11. The schematic representation of FIGURE 12 further includes at least one rendering, including a tissue rendering 1202, a signs rendering 1204 and/or a segmented liver rendering 1206. The tissue rendering 1202 shows the segmented tissue including the liver, the spleen, the gallbladder, etc. The signs rendering 1204 shows the segmented elements that are signs of disease of the primary tissue. The segmented liver rendering 1206 shows the segmented liver. In this example, the segmented liver rendering 1206 shows the Couinaud segmentation.

The schematic representation summarizes a health state of a liver of a subject. For a trained radiologist, a few seconds of examining the schematic representation is likely enough to determine whether further analysis of the liver is required. This schematic representation can also be included in clinical reports to share with other radiologists or to summarize studies, facilitating communication and collaboration. The schematic representation considers both primary and secondary signs of liver disease in primary tissue of interest, and searches for indications of the liver disease in other organs and/or other areas. The approach can be used for screening and can process image data acquired for reasons unrelated to the liver (e.g., an abdomen scan), potentially triggering further analysis and early disease detection.

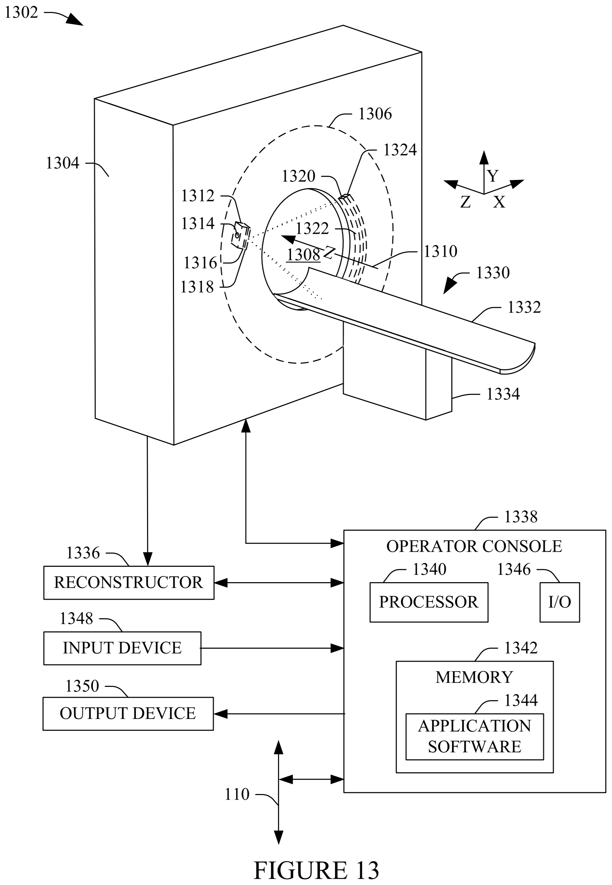

As discussed herein, the approach described can process image data from same and/or different modality concurrently or separately, and examples of the image data include one or more of CT, MR, PET, SPECT, and/or other image data. With the user-case scenario presented in connection with FIGURES 11 and 12, the modality of interest was CT. FIGURE 13 diagrammatically illustrates a non-limiting example of an imaging system 1302 configured for CT imaging.

The imaging system 1302 includes a gantry 1304. In some instances, the gantry 1304 is configured to tilt. The imaging system 1302 further includes a rotating frame 1306. The rotating frame 1306 is rotatably supported in the gantry 1304, e.g., via a bearing (e.g., a slip ring) or the like, and is configured to rotate around an examination region 1308 about a rotational or z-axis 1310, which extends through a center of rotation / a center of the examination region 1308 (i.e., an isocenter). A gantry controller (not visible) is configured to control rotation of the rotating frame 1306 and, if configured to tilt, tilting of the gantry 1304.

An X-ray source assembly 1312 is supported by the rotating frame 1306 and rotates in coordination with the rotating frame 1306. The X-ray source assembly 1312 includes an X-ray source 1314 such as an X-ray tube. The X-ray source 1314 is configured to emit X-ray radiation having an energy in the X-ray diagnostic range (e.g., 20 keV to 150 keV). The X-ray source assembly 1312 may further include or is coupled to a filter 1316 that characterizes an X-ray radiation dose profile and/or a collimator 1318 that shapes the X-ray radiation to form a generally fan, wedge, cone, etc. shaped beam that traverses the examination region 1308. An X-ray controller (not visible) is configured to control components of the X-ray assembly 1312 such as X-ray radiation emission of the X-ray source 1314, the collimator 1318, etc.

An X-ray radiation sensitive detector array 1320 includes a one-dimensional (1-D) or two-dimensional (2-D) array of rows of X-ray radiation sensitive detector elements 1322 and is supported by the rotating frame 1306 along an arc opposite the X-ray source 1314, across the examination region 1308. Each of the X-ray radiation sensitive detector elements 1322 is in electrical communication with a data acquisition system (DAS) 1324. The X-ray radiation sensitive detector elements 1322 include an indirect conversion detector such as a scintillator / photodiode detector and/or a direct conversion detector such as a Cadmium Telluride (CdTe), a Cadmium Zinc Telluride (CZT), etc. detector. A DAS controller (not visible) controls the X-ray radiation sensitive detector array 1320.

A subject/object support 1330 includes a tabletop 1332 moveably coupled to a frame/base 1334. In one instance, the tabletop 1332 is slidably coupled to the frame/base 1334 via a bearing or the like, and a drive system (not visible) including a controller, a motor, a lead screw, and a nut (or other drive system) translates the tabletop 1332 along the frame/base 1334 into and out of the examination region 1308. The tabletop 1332 is configured to support an object or subject in the examination region 1308 for loading, scanning, and/or unloading the subject or object. A table controller (not visible) controls the drive system.

For an axial scan, the tabletop 1332 is positioned at a static position for each integration period and moves between integration periods. For a helical scan, the rotating frame 1306 rotates in coordination with the tabletop 1332 moving along the Z-axis 1310, and active X-ray detector elements 1322 of the X-ray radiation sensitive detector array 1320 detect X-ray radiation over consecutive arc segments (integration periods) each revolution and generate respective signals. For each arc segment, the data acquisition system (DAS) 1324 processes each signal and generates projection data.

A reconstructor 1336 reconstructs the projection data and generates volumetric three-dimensional (3-D) image data for a helical scan and/or individual axial two-dimensional (2-D) images for an axial step and shoot scan (which can be used in combination to generate volumetric image data). The volumetric image data and/or 2-D slices thereof, and/or the individual axial images can be visually presented, filmed, etc. Examples of suitable reconstruction algorithms include filtered back projection (FBP), advanced statistical iterative reconstruction (ASIR), conjugate gradient (CG), maximum likelihood expectation maximization (MLEM), model-based iterative reconstruction (MBIR), and/or other reconstruction algorithm.

With a non-spectral configuration, the X-ray source 1314 includes a single broadband X-ray tube that emits polychromatic radiation, e.g., 40 keV to 120 keV at 1320 kVp. In another example, the imaging system 1302 is configured for spectral imaging. With such a configuration, the X-ray source 1314 may be configured to switch between at least two different kVp values, include multiple X-ray tubes, etc., and/or the detector array 1320 may include multiple layers, each configured to detect a different energy, etc., where the projection data can be decomposed into photoelectric effect and Compton scattering components, and the reconstructor 1336 can reconstruct projection data for different energy bands.

A computing system serves as an operator console 1338 of the system 1302. The computing system 1338 may include a computer, a workstation, etc. The computing system 1338 further includes at least one processor 1340 such as a µP, a CPU, a GPU, etc., and a computer readable storage medium 1342 ("MEMORY"), which includes non-transitory medium and excludes transitory medium (signals, carrier waves, and the like). The memory 1342 includes application software 1344, which allows a user to set up and start a scan, view image data, transfer image data over the network 110, etc. The computing system 1338 further includes I/O 1346, an input device 1348, and an output device 1350.

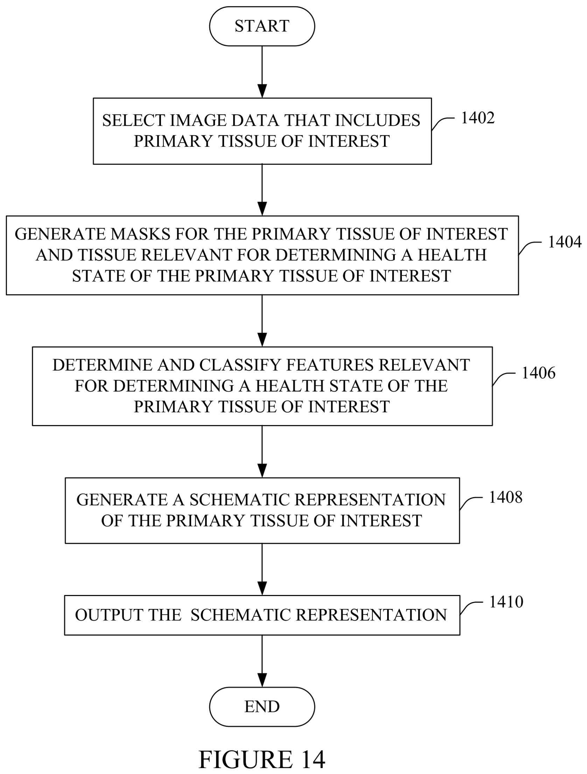

FIGURE 14 illustrates a non-limiting example of a flow chart for generating a schematic representation that provides a summary of a health state of tissue of interest, in accordance with an aspect herein. It is to be appreciated that the ordering of the acts in the method is not limiting. As such, other orderings are contemplated herein. In addition, one or more acts may be omitted, and/or one or more additional acts may be included.

At 1402, the image data selector 202 accesses the data repository 106 and selects one or more sets of image data stored in the data repository 106 for processing by the segmentation mask generator 204, as described herein and/or otherwise. At 1404, the segmentation mask generator 204 processes the one or more sets of image data, each with a set of corresponding algorithms and outputs segmentation masks, as described herein and/or otherwise. At 1406, the feature determiner 206 processes, the masks generated by the segmentation mask generator 204 and derives and classifies metrics, as described herein and/or otherwise.

At 1408, the schematic representation generator 208 processes the masks and the derived metrics and generates a schematic representation that summarizes the information in the image data, the masks and the metrics, as described herein and/or otherwise. In one instance, the summary includes a graphical representation of the primary tissue of interest and the relevant other tissue generated from the image data. In some instances, two and/or three dimensional measurements of the tissue are also included in the schematic representation. In some instances, the derived and classified metrics are also included with the schematic representation. In some instances, renderings of the tissue are also included in the schematic representation.

At 1410, the schematic representation module 116 outputs the schematic representation, as described herein and/or otherwise. An example of a schematic representation generated from CT image data of the liver is described in connection with FIGURE 11. Another example of a schematic representation generated from CT image data of the liver is described in connection with FIGURE 12. The schema can be incorporated into a report, printed, archived, communicated to another device, etc.

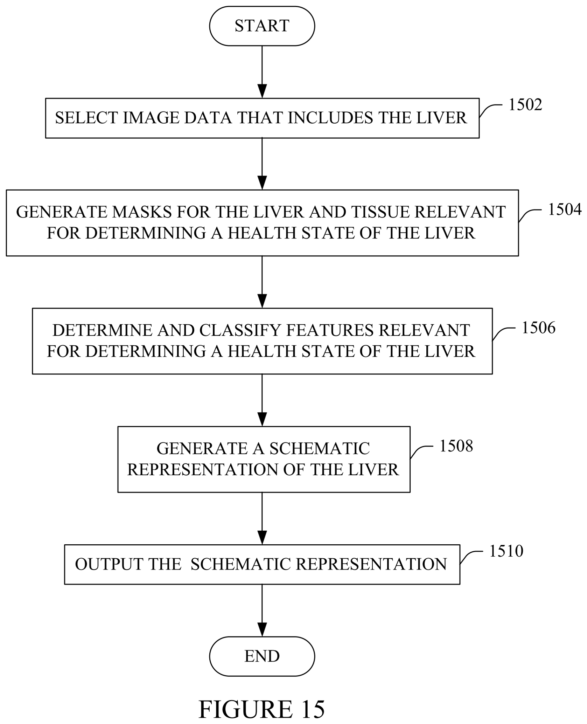

FIGURE 15 illustrates a non-limiting example of a flow chart for generating a schematic representation that provides a summary of a health state of the liver for a particular subject, in accordance with an aspect herein. It is to be appreciated that the ordering of the acts in the method is not limiting. As such, other orderings are contemplated herein. In addition, one or more acts may be omitted, and/or one or more additional acts may be included.

At 1502, the image data selector 202 accesses the data repository 106 and selects one or more sets of image data that includes the liver from the data repository 106 for processing by the segmentation mask generator 204, as described herein and/or otherwise. For example, in one instance the image data selector 202 selects each set of image data at least based on a particular medical imaging modality, the liver as the primary organ, and the relevant other organs including the spleen, the kidneys, the pancreas, etc. In another instance, the image data selector 202 further filters the selection to contrast material enhanced image data.

At 1504, the segmentation mask generator 204 processes the one or more sets of image data, each with a set of corresponding algorithms and outputs segmentation masks, as described herein and/or otherwise. Again, the processing of the one or more sets of image data can be performed serially and/or in parallel. Where a set of image data includes subsets of image data from different studies for the subject, the segmentation mask generator 204 independently processes the subsets of image data for the subject. For each set (and/or subset) of image data processed, the segmentation mask generator 204 generates and outputs a plurality of masks such as an organ segmentation mask, a sub-organ segmentation mask, and a relevant other tissue mask.

For example, the segmentation mask generator 204 employes an algorithm configured to segment the liver in CT image data and generate a liver mask. The segmentation mask generator 204 employes an algorithm configured to segment the liver segmentation into sub-segments and generate a mask of the sub-segments. A non-limiting segmentation approach includes a Couinaud segment, which segments the liver into eight sub-segments. The segmentation mask generator 204 further employes an algorithm configured to segment elements that are signs of disease of the liver such as the right hepatic notch, portal veins, the gallbladder, liver surface nodularity, the ligament falciform, collateral vessels, ascites, and/or the perihilar portal space, and/or other tissue and generate masks thereof.

At 1506, the feature determiner 206 processes the masks generated by image data processor 202 and derives and classifies the metrics, as described herein and/or otherwise. For instance, the feature determiner 206 determines measurements such as size, shape, etc. of different parts of the liver and derive metrics based on ratios, comparisons, etc. of the measurements. By way of non-limiting example, where the liver is segmented based on the Couinaud segmentation (which includes eight segments), the feature determiner 206 can define a feature referred to as Left Liver hypertrophy and compute a value for the feature through a ratio of the segments 1 and 2 of the Couinaud segmentation versus a total liver volume. The value for the Left Liver hypertrophy feature can be classified based on information from a pool of information corresponding to healthy livers, and, in some instance, further classified based on information from a pool of information corresponding to unhealthy livers.

At 1508, the schematic representation generator 208 processes the masks and the derived metrics, and generates a schematic representation that summarizes a health of the liver from the information in the masks and metrics, as described herein and/or otherwise. The summary includes a graphical representation of the liver and the relevant other tissue generated from the image data. The location and size of the liver and the spleen, the kidneys, etc. reflect the actual location and size of the liver for a particular slice/image of interest of the image and an accumulation of locations and sizes of the relevant other tissue derived from a set of slices/images in a window around the particular slice/image. In one instance, one or more of the sub-schema for the liver and the spleen, the kidneys, etc. will include one or more measurements.

In another instance, the schematic representation generator 208 further processes the derived and classified metrics, and the schematic representation further summarizes the information in the derived and classified metrics, as described herein and/or otherwise. For example, in one instance the summary includes a list of each metric and maps each metric to a corresponding health state. By way of non-limiting example, an example table includes a two-by-two table with a first column that lists an identification of each derived metric and a second column that lists the derive health states, where each row includes a derived metric in the first column (e.g., row 1, column 1) and a corresponding health state in the second column (e.g., row 1, column 2). The table can further be delineated to group derived metrics across categories of interest.

In some instances, the schematic representation generator 208 further processes the set of image data and/or the masks and generates at least one rendering, as described herein and/or otherwise. For example, in one instance the schematic representation generator 208 processes such information and generates a rendering of the liver. In this instance, the schematic representation generator 208 can label different sections of the liver via textual and/or graphical indicia. Additionally, or alternatively, the schematic representation generator 208 processes such information and generates a rendering of the other relevant tissue. Likewise, the schematic representation generator 208 can label different sections of the other spleen, the kidneys, the pancreas, etc. via textual and/or graphical indicia. Additionally, or alternatively, the schematic representation generator 208 processes such information and generates a rendering of the elements characteristic of a liver disease, including the right hepatic notch, portal veins, the gallbladder, liver surface nodularity, the ligament falciform, collateral vessels, ascites, and/or the perihilar portal space, and/or other tissue and generate masks of tissue reflective of the health state of the liver.

At 1510, the schematic representation module 116 outputs the schematic representation of the liver, as described herein and/or otherwise. An example of a schematic representation generated from CT imaged data of the liver is described in connection with FIGURE 11. Another example of a schematic representation generated from CT imaged data of the liver is described in connection with FIGURE 12. The schema can be incorporated into a report, printed, archived, communicated to another device, etc.

The above can be implemented by way of computer readable instructions, encoded, or embedded on the computer readable storage medium, which, when executed by a computer processor, cause the processor to carry out the described acts or functions. Additionally, or alternatively, at least one of the computer readable instructions is carried out by a signal, carrier wave or other transitory medium, which is not computer readable storage medium.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include such additional elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

The various embodiments and/or components, for example, the modules, or components and controllers therein, also may be implemented as part of one or more computers or processors. The computer or processor may include a computing device, an input device, a display unit and an interface, for example, for accessing the Internet. The computer or processor may include a microprocessor. The microprocessor may be connected to a communication bus. The computer or processor may also include a memory. The memory may include Random Access Memory (RAM) and Read Only Memory (ROM). The computer or processor further may include a storage device, which may be a hard disk drive or a removable storage drive such as a floppy disk drive, optical disk drive, and the like. The storage device may also be other similar means for loading computer programs or other instructions into the computer or processor.

As used herein, the term "computer" or "module" may include any processor-based or microprocessor-based system including systems using microcontrollers, reduced instruction set computers (RISC), application specific integrated circuits (ASICs), logic circuits, and any other circuit or processor capable of executing the functions described herein. The above examples are exemplary only, and are thus not intended to limit in any way the definition and/or meaning of the term "computer". The computer or processor executes a set of instructions that are stored in one or more storage elements, in order to process input data. The storage elements may also store data or other information as desired or needed. The storage element may be in the form of an information source or a physical memory element within a processing machine.

The set of instructions may include various commands that instruct the computer or processor as a processing machine to perform specific operations such as the methods and processes of the various embodiments of the invention. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to operator commands, or in response to results of previous processing, or in response to a request made by another processing machine.

As used herein, the terms "software" and "firmware" are interchangeable, and include any computer program stored in memory for execution by a computer, including RAM memory, ROM memory, EPROM memory, EEPROM memory, and non-volatile RAM (NVRAM) memory. The above memory types are exemplary only, and are thus not limiting as to the types of memory usable for storage of a computer program.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the various embodiments of the invention without departing from their scope. While the dimensions and types of materials described herein are intended to define the parameters of the various embodiments of the invention, the embodiments are by no means limiting and are exemplary embodiments. Many other embodiments will be apparent to those of skill in the art upon reviewing the above description.

This written description uses examples to disclose the various embodiments of the invention, including the best mode, and also to enable any person skilled in the art to practice the various embodiments of the invention, including making and using any devices or systems and performing any incorporated methods. The patentable scope of the various embodiments of the invention is defined by the claims, and may include other examples that occur to those skilled in the art. Such other examples are intended to be within the scope of the claims if the examples have structural elements that do not differ from the literal language of the claims, or if the examples include equivalent structural elements with insubstantial differences from the literal languages of the claims.

Embodiments of the present disclosure shown in the drawings and described above are example embodiments only and are not intended to limit the scope of the appended claims, including any equivalents as included within the scope of the claims. Various modifications are possible and will be readily apparent to the skilled person in the art. It is intended that any combination of non-mutually exclusive features described herein are within the scope of the present disclosure. That is, features of the described embodiments can be combined with any appropriate aspect described above and optional features of any one aspect can be combined with any other appropriate aspects. Similarly, features set forth in dependent claims can be combined with non-mutually exclusive features of other dependent claims, particularly where the dependent claims depend on the same independent claim. Single claim dependencies may have been used as practice in some jurisdictions that require them, but this should not be taken to mean that the features in the dependent claims are mutually exclusive.

## Claims

1. A computer-implemented method, comprising:
obtaining a first mask of primary tissue of interest segmented from image data of a subject, wherein a health state of the primary tissue of interest is under evaluation;
obtaining a second set of masks of other tissue indicative of the health state of the primary tissue of interest from the image data of the subject;
obtaining a third set of masks of elements that are signs of disease of the primary tissue of interest from the image data of the subject; and
generating a schematic representation of the health state of the primary tissue of interest of the subject with the image data, the first mask, the second set of masks and the third set of masks, wherein the schematic representation provides a summary of health related information about the health state of the primary tissue of interest from the image data.

2. The computer-implemented method of claim 1, further comprising:
determining at least one measurement for at least one tissue in at least one of the first mask, the second set of masks and the third set of masks; and
including the at least one measurement for the at least one tissue in the schematic representation.

3. The computer-implemented method of claim 1, further comprising:
obtaining a sub-segmentation mask of the first mask;
deriving metrics based on at least one of the sub-segmentation mask, the first mask, the second set of masks and the third set of masks;
classifying each of the derived metrics as indicating healthy or not healthy tissue; and
including the derived metrics and corresponding classifications with the schematic representation.

4. The computer-implemented method of claim 3, further comprising:
sub-classifying each derived metric classified as not healthy tissue across two or more or a continuum of sub-classifications.

5. The computer-implemented method of claim 1, further comprising:
generating the schematic representation based on a set of rules that identifies a set of images slices in the image data for each tissue in the schematic representation.

6. The computer-implemented method of claim 5, where the set of rules includes:
incorporating the primary tissue of interest from a predetermined image slice of the image data into the schematic representation.

7. The computer-implemented method of claim 6, where the set of rules includes:
incorporating at least one tissue of the other tissue from an image slice of the image data that includes a largest dimension of the at least one tissue into the schematic representation.

8. The computer-implemented method of claim 6, where the set of rules includes:
incorporating an accumulation of at least one element of the elements through the image data into the schematic representation.

9. The computer-implemented method of claim 6, where the set of rules includes:
incorporating an accumulation of at least one element of the elements from a predetermined set of image slices about the image slice into the schematic representation.

10. The computer-implemented method of claim 6, where the set of rules includes:
incorporating a contour of the subject from the image slice into the schematic representation.

11. The computer-implemented method of claim 9, further comprising:
obtaining a rendering of at least one of:
the primary tissue of interest with the other tissue;
the elements, and
sub-portions of the primary tissue of interest from the sub-segmentation;
including the rendering with the schematic representation.

12. The computer-implemented method of claim 1, further comprising:
including the schematic representation with a medical report for the subject.

13. A system (102), comprising:
a display device (122);
a memory (114) configured to store computer-readable instructions for generating a schematic representation of a health state of a primary tissue of interest of a subject based on image data of the subject;
at least one processor (112) configured to execute the computer-readable instructions, which causes the at least one processor to:
obtain a first mask of the primary tissue of interest segmented from the image data;
obtain a second set of masks of other tissue indicative of the health state of the primary tissue of interest from the image data of the subject;
obtain a third set of masks of elements that are signs of disease of the primary tissue of interest from the image data of the subject; and
generate the schematic representation with the image data, the first mask, the second set of masks and the third set of masks, wherein the schematic representation provides a summary of health related information about the health state of the primary tissue of interest from the image data.

14. The system of claim 13, wherein the at least one processor is further configured to:
incorporate the primary tissue of interest from an image slice of the image data corresponding to a predetermined functional state of the primary tissue of interest into the schematic representation;
incorporate at least one tissue of the other tissue from an image slice of the image data that includes a largest dimension of the at least one tissue into the schematic representation;
incorporate an accumulation of at least one element of the elements through the image data into the schematic representation;
incorporate an accumulation of at least one element of the elements from a predetermined set of image slices about the image slice into the schematic representation; and
incorporate a contour of the subject from the image slice into the schematic representation.

15. The system of claim 14, wherein the at least one processor is further configured to:
determine at least one measurement for at least one tissue in at least one of the first mask, the second set of masks and the third set of masks;
include the at least one measurement with the schematic representation;
obtain a sub-segmentation mask of the first mask;
derive metrics based on at least one of the sub-segmentation mask, the first mask, the second set of masks and the third set of masks;
classify each of the derived metrics as indicating healthy or not healthy tissue;
include the derived metrics and corresponding classifications with the schematic representation;
obtain a rendering of at least one of:
the primary tissue of interest with the other tissue;
the elements, and
sub-portions of the primary tissue of interest from the sub-segmentation; and include the rendering with the schematic representation.
